# EUROPEAN PATENT SPECIFICATION

(11) **EP 2 981 242 B1**
(45) Date of publication and mention of the grant of the patent: **19.07.2017**
(21) Application number: 14715250.8
(22) Date of filing: 28.03.2014
(51) Int. Cl.: A61K 8/04, A61K 8/20, A61K 8/26, A61K 8/37, A61Q 15/00

(54) **METHOD FOR THE MANUFACTURE OF ANHYDROUS COSMETIC COMPOSITIONS**
VERFAHREN ZUR HERSTELLUNG VON ANHYDRISCHEN KOSMETIKZUSAMMENSETZUNGEN
PROCÉDÉ POUR LA FABRICATION DE COMPOSITIONS COSMÉTIQUES ANHYDRES

(30) Priority: 03.04.2013 EP 13162122
(43) Date of publication of application: 10.02.2016
(73) Proprietor: Unilever PLC, London, EC4Y 0DY (GB); Unilever N.V., 3013 AL Rotterdam (NL)
(72) Inventor: BAKER, Michael, Richard, Wirral Merseyside CH63 3JW (GB); FLETCHER, Neil, Robert, Wirral Merseyside CH63 3JW (GB); FRANKLIN, Kevin, Ronald, Wirral Merseyside CH63 3JW (GB); STOCKTON, Joanne, Elizabeth, Wirral Merseyside CH63 3JW (GB)
(74) Representative: Whaley, Christopher
(86) International application number: PCT/EP2014/056347
(87) International publication number: WO 2014/161792

(56) References cited:
- EP-A1- 1 974 716
- EP-A1- 2 574 327
- WO-A2-2013/013902

## Description

The present invention is in the field of cosmetic compositions, in particular the manufacture of anhydrous cosmetic compositions.

A variety of cosmetic compositions have been marketed for many years. They serve many functions, particularly following application to the surface of the body. Such compositions frequently add fragrance to the surface, i.e. skin of the human body. Particular cosmetic compositions known as antiperspirant compositions also reduce perspiration of the human body and these are most commonly applied to the underarm regions.

Calcium chloride has been used in cosmetic compositions for many years and for a variety of purposes. Sometimes, it is used as a dried powder, it which state its formulation can be somewhat challenging because of its hygroscopicity.

Alum salts have also been used in cosmetic compositions for many years and for a variety of purposes.

EP 1,974,716 A (Sara Lee, 2007) and WO 08/120976 (Sara Lee, 2008) disclose cosmetic compositions, for instance deodorant compositions, comprising at least partially dehydrated aluminium sulphate and a carrier liquid other than water.

US 133,430 (John Gamgee, 1872) discloses the manufacture of a deodorising powder by mixing/grinding together aluminium sulphate (sulphate of alumina or "alum") and calcium chloride.

WO 2013/013902 A2 (L'Oréal, 2013) discloses fragranced aerosol antiperspirant compositions comprising calcium chloride, alum and isopropyl palmitate, which are free of dipropyleneglycol; however, this document proposes keeping the calcium salt separate from the aluminium salt during storage.

EP 2,574,327 A1 (Unilever, 2013) discloses fragranced antiperspirant compositions comprising calcium chloride and alum, but does not discuss the importance of any solvent present in the fragrance.

Other publications, such as US 5,955,065 (Gillette, 1999), have described the use of water soluble calcium salts to enhance the performance of conventional antiperspirant actives. The chemistry described in such publications involves the enhancement of peaks 3 and 4 on the HPLC trace of such antiperspirant actives. The species responsible for these peaks are not generated in the methods described herein and the chemistry behind the present invention is entirely different (*vide infra*).

In formulating cosmetic compositions comprising dried calcium chloride and fragrance in aerosol compositions, the present inventors have observed a problem that they believe has not previously been identified. They have found that the stability of such compositions is highly dependent upon the method of manufacture and particularly the nature of the fragrance-solvent pre-mix used in such manufacture. They have found the problem to be particularly severe in aerosol compositions also comprising a dried alum salt.

An objective of the present invention is to provide effective means of manufacture for cosmetic compositions comprising a fragrance, a solvent for said fragrance, dried calcium chloride, a propellant, and dried alum salt.

In a first aspect of the present invention, there is provided a method of manufacture of an anhydrous cosmetic composition comprising the mixing of dried calcium chloride and a dried alum salt with liquid carrier material and fragrance comprising a solvent to give a base composition, followed by gassing of the base composition with a propellant to give an aerosol composition, wherein the fragrance and the solvent therein are pre-mixed and this mixture:
i). comprises one or more components having a logP of greater than 5.5 and a vapour pressure at 25°C of less than 0.001 mm Hg (0.1333 Pa);
ii). does not comprise dipropyleneglycol at 3% or greater of this mixture; the liquid carrier material including all the liquid components of the composition, excluding any liquid fragrance and solvent comprised therein.
Herein, the term "dried" should be understood to include materials having both crystalline and amorphous states of matter. Such powders have a water content that is reduced from that of the most hydrated natural salt of the particular salt being used.

Herein, the term "dehydrated" when used with reference to a salt should be understood to refer to a salt that has a water content that is reduced from that of the most hydrated natural salt of the particular salt being used.

Herein, the term "anhydrous" should be understood to mean having less than 2% by weight of free water. Preferably, anhydrous compositions have less than 1% by weight free water and more preferably less than 0.5%.

Herein "free water" is water other than the water of hydration associated with any particular component. Dehydrated alum salts and water soluble calcium salts are available as free flowing powders which typically have some water associated with them and this is typically water of hydration.

Herein, the term "liquid" should be understood to refer to a state of matter at ambient temperature and pressure, by which is meant 20°C and 1 atmosphere.

Herein, the term "oil" should be understood to refer to a substance immiscible with water that is a liquid at ambient temperature and pressure.

Herein, the term "predominately" should be understood to refer to an amount greater than 50% by weight of the mixture referred to.

Herein, "pre-mix" should be understood to mean the prep-mixed fragrance and solvent used in the manufacturing method of the invention.

Herein, all percentages (%) should be understood to be percentages by weight (% w/w), unless otherwise stated.

It is preferred that anhydrous compositions have a total water content (including water of hydration associated with components therein) of less than 10% by weight, and more preferably less than 5%.

The fragrance employed in the present invention comprises a solvent. Commercial fragrances are often sold with one or more solvents present. In general, such solvents are of relatively low odour. The solvent may also be of relatively low volatility, preferably having a vapour pressure at 25°C of less than 0.001 mm Hg (0.1333 Pa). The solvent serves to dilute, dissolve or co-dissolve with the fragrance components. The solvent is usually different to the liquid carrier material referred to in the first aspect of the invention *et seq.*

In the manufacturing method of the present invention, it should be understood that the fragrance and its solvent are used as a pre-mix, i.e. the fragrance is dissolved in its solvent prior to its mixing with the other components of the composition being manufactured.

The manufacturing method of the present invention requires that the fragrance and the solvent therein are pre-mixed and that this mixture comprises one or more components having a logP of greater than 5.5 and a vapour pressure at 25°C of less than 0.001 mm Hg (0.1333 Pa) and does not comprise dipropyleneglycol at 3% or greater of this mixture. The reference to the fragrance and solvent being "pre-mixed" means that the process must involve these components being mixed with one another independently of their mixing with any of the other components of the composition.

The importance of the pre-mix component having a logP of greater than 5.5 and a vapour pressure at 25°C of less than 0.001 mm Hg (0.1333 Pa) is that is helps dilute the fragrance without causing stability problems and without itself harming the hedonics of the fragrance too greatly. Diluting the fragrance in this way can aid dosing of the fragrance and co-solubilisation of the fragrance components.

Components of the pre-mix having a logP of greater than 5.5 and a vapour pressure at 25°C of less than 0.001 mm Hg are preferably present at a level of 10% or greater by weight of said pre-mix and more preferably at a level of 20% or greater by weight of said pre-mix.

The importance of the pre-mix not comprising dipropyleneglycol at 3% or greater lies in the great sensitivity of the process and resulting composition to excessive dipropyleneglycol. It is important that this component is kept to a relatively low level in both the manufacture and final composition. It is preferred that the pre-mixed fragrance and solvent does not comprise dipropyleneglycol at 1% or greater.

The component or components of the pre-mix having a logP of greater than 5.5 and a vapour pressure at 25°C of less than 0.001 mm Hg are preferably comprised predominately of isopropyl esters of myristic acid, lauric acid, and/or palmitic acid. By this it meant that these isopropyl esters comprise in total greater than 50% by weight of the components of the pre-mix having these logP and vapour pressure characteristics. It is particularly preferred that these isopropyl esters comprise 70% or greater and especially preferred that they comprise 90% or greater by weight of such components.

The most preferred component of the pre-mix having a logP of greater than 5.5 and a vapour pressure at 25°C is isopropyl myristate (IPM). IPM preferably comprises 50% or greater, more preferably 70% or greater, and most preferably 90% or greater by weight of the components of the pre-mixed fragrance and solvent having a logP of greater than 5.5 and a vapour pressure at 25°C.

Surprisingly, the present inventors found that compositions comprising dried alum salts and dried calcium chloride prepared in accordance with the method of the invention gave excellent antiperspirant compositions. Such compositions were found to give good antiperspirant benefits when applied to the surface of the human body. Such compositions were also found to have surprising stability benefits compared with such compositions prepared other ways.

The term alum salt as used in the present description means aluminium sulphate (sometimes called "alum") or any double sulphate of aluminium and a univalent metal ion selected from potassium, sodium, or ammonium. It does not include alum salts that are double sulphates of a univalent metal and a trivalent metal other than aluminium, such as chromium (III) or iron (III).

Suitable potassium alum, ammonium alum, sodium alum and aluminium sulphate fall within the general formulae:

M(i)Al(SO₄)₂ or Al₂(SO₄)₃

wherein M(i) is K⁺, Na⁺, NH₄⁺ or mixture thereof.

Particularly suitable alum salts are ammonium and potassium alum, in particular potassium alum.

Alum salts used in the present invention have a reduced content of water, that is to say, they are at least partially dehydrated. They may alternatively be described as dried (*vide supra*).

Preferred alum salts have a water content of less than 28% by weight and especially preferred alum salts have a water content of less than 20% by weight. When water is present, it is typically present as water of hydration. The alum salt is typically milled to give it a reduced particle size. In preferred embodiments, the particle size distribution of the alum salt is such that its D50 is less than 75 microns and more preferably less than 50 microns. The particle size distribution of the alum salt is preferably such that less than 5% and more preferably less than 1 % by weight of the particles have a particle size of greater than 120 microns.

The particle size distribution of the alum salt may advantageously be measured using a light scattering method on a Malvern Mastersizer 2000. The powder is dispersed in silicone fluid (cyclopentasiloxane) and the results are analysed assuming a particle refractive index of 1.55 and imaginary refractive index of 0.001.

The calcium chloride is dried. It is preferred that the calcium chloride has a water content of 25% or less and particularly preferred that the water content is 8% or less. Suitable calcium chloride salts for such compositions include calcium chloride dihydrate and anhydrous calcium chloride, with anhydrous calcium chloride being preferred. It should be noted, however, that anhydrous calcium chloride as obtained from some suppliers can include up to about 14% by weight of water of hydration.

The calcium chloride used in the present invention is typically milled to give it a reduced particle size. In preferred embodiments, the particle size distribution of the calcium chloride is such that its D50 is less than 100 microns, more preferably less than 75 microns and most preferably less than 50 microns. The particle size distribution of the calcium chloride is preferably such that less than 5% and more preferably less than 1% by weight of the particles have a particle size of greater than 120 microns.

The particle size distribution of the alum salt may advantageously be measured using a light scattering method on a Malvern Mastersizer 2000. The powder is dispersed in silicone fluid (cyclopentasiloxane) and the results are analysed assuming a particle refractive index of 1.55 and imaginary refractive index of 0.001. Employing dried alum salt in the present invention, the antiperspirancy benefit observed is hypothesised to be related to the following chemical reaction:

KAl(SO₄)₂ + 2CaCl₂ -> 2CaSO4↓ + KCI + AlCl₃

Or

Al₂(SO₄)₃ + 3CaCl₂ -> 3CaSO4↓ + 2AlCl₃

In the top equation, the potassium ion (K⁺) may be substituted by sodium (Na⁺) or ammonium (NH₄⁺).

The stoichiometry of the above equations requires one mole of alum to two moles of calcium chloride in the first and one mole of alum to three moles of calcium chloride in the second. These equations set the basis for the preferred ratios of these components. It is preferred that the molar quantity of calcium chloride exceeds the molar quantity of alum salt. It is also preferred that the quantity of calcium chloride at least matches that stoichiometrically required by the above equations, relative to the amount and type of alum present. This means that it is preferred that the molar ratio of calcium chloride to alum salt is at least 2:1.

In compositions comprising calcium chloride and sodium, potassium or ammonium alum as the major alum salt present, the molar ratio of calcium chloride to alum salt is preferably from 1:1 to 5:1, more preferably from 3:2 to 3:1, and most preferably about 2:1.

In compositions comprising calcium chloride and aluminium sulphate as the major alum salt present, the molar ratio of calcium chloride to alum salt is preferably from 2:1 to 6:1, more preferably from 5:2 to 4:1, and most preferably about 3:1.

It is important that the reaction indicated above only occurs to a minimal extent before the components are delivered to the surface of the human skin. Premature reaction results in a physical state of matter which tends not to deliver the desired benefits; indeed, it is commonly extremely difficult to even apply said matter to the desired location.

The chemical reaction involved may only occur when the ions making up the reactants have sufficient mobility. In certain preferred embodiments of the present invention, this mobility typically arises when the reactants dissolve in aqueous body fluids found on the surface of the human body.

An essential component of the invention is a liquid carrier material. The present inventors have found it preferable that a large proportion (50% or more) of the liquid carrier material must be comprised of oils having a HSP of 18.02 (MPa)^{0.5} or less. Preferably, the liquid carrier material comprises 70% or more of such oils and more preferably 80% or more. In addition, the liquid carrier material must not comprise more than modest amounts of liquid components having a less hydrophobic character. It has been found that the less hydrophobic a liquid component is, the less can it be tolerated. Thus, the liquid carrier material must comprise 30% or less of liquids having a HSP of 18.25 (MPa)^{0.5} or greater; 20% or less of liquids having a HSP of 18.61 (MPa)^{0.5} or greater; and 10% or less of liquids having a HSP of 23.20 (MPa)^{0.5} or greater.

It will be understood that the liquid carrier material may only comprise liquids having a HSP of greater than 18.02 (MPa)^{0.5} in an amount of less than 50%, preferably less than 30%, and more preferably less than 20% by weight.

The liquid carrier material should be considered to include all the liquid components of the composition excluding any liquid fragrance and solvent comprised therein. HSPs for many cosmetic liquids can be obtained from the literature, such as from C. D. Vaughan, J. Soc. Cosmet. Chem. (1985), 36, 319-333 or from Table 1 in US 2010/0047296 (Henkel). For liquids where literature values have not been reported they may be calculated using methods described in the above reference or the methods described by S. W. van Krevelen in Properties of Polymers, p200-225, Elsevier, (1990).

Suitable oils having a HSP of 18.02 (MPa)^{0.5} or less are triethylhexanoin, isopropyl myristate, isopropyl palmitate, diisopropyl adipate, hexadecane, C12-15 alkyl benzoate, dioctyl ether, white mineral oil, dimethicone fluid, phenyl trimethicone and cyclomethicones such as cyclopentasiloxane. In preferred embodiments, the liquid carrier material comprises 80% or more by weight of oils selected from this list.

The liquid carrier material preferably comprises oils having a HSP of 16.36 (MPa)^{0.5} or less. In preferred embodiments, the liquid carrier material comprises 50% or more by weight of oils having a HSP of 16.36 (MPa)^{0.5} or less.

Suitable oils, having a HSP of 16.36 (MPa)^{0.5} or less are hexadecane, C12-15 alkyl benzoate, isopropyl palmitate, dioctyl ether, white mineral oil, dimethicone fluid, phenyl trimethicone and cyclomethicones such as cyclopentasiloxane. In particularly preferred embodiments, the liquid carrier material comprises 85% or more by weight of oils selected from this list.

Preferred components of the liquid carrier material also perform an addition function; particularly preferred functions being to act as emollients and/or masking liquids.

Preferred components of the liquid carrier material are anhydrous, as described hereinabove. Preferably, they contain less than 2%, more preferably less than 1% and most preferably less than 0.5% by weight free water.

The liquid carrier material is preferably included in the composition at a level of 1-90%, more preferably at from 10 to 80% and most preferably at from 20 to 70% by weight of the composition, excluding the propellant that is also present in the final composition.

Other components may also be included in compositions used in accordance with the invention.

Additional antiperspirant actives may also be included.

The total amount of antiperspirant actives, including dehydrated alum salt and calcium chloride, incorporated in a composition is preferably from 0.5-50%, particularly from 1 to 30% and especially from 2% to 26% of the weight of the composition.

Antiperspirant actives used in addition to the alum salt and calcium chloride combination are often selected from astringent active salts, including in particular aluminium and aluminium/zirconium salts. Preferred additional antiperspirant actives are aluminium halides and halohydrate salts, such as chlorohydrates.

Suitable aluminium halohydrates are defined by the general formula Al₂(OH)ₓQ_{y}.wH₂0 in which Q represents chlorine, bromine or iodine, x is variable from 2 to 5 and x + y = 6 while wH₂O represents a variable amount of hydration. Especially effective aluminium halohydrate salts are known as activated aluminium chlorohydrates and are made by methods known in the art.

The proportion of solid antiperspirant salt in a suspension composition normally includes the weight of any water of hydration and any complexing agent that may also be present in the solid active.

Additional deodorant actives may also be included. When employed, the level of incorporation is preferably from 0.01% to 3% and more preferably from 0.03% to 0.5% by weight. Preferred deodorant actives are those that are more efficacious than simple alcohols such as ethanol. Examples include quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred are polyhexamethylene biguanide salts (also known as polyaminopropyl biguanide salts), an example being Cosmocil CQ available from Arch Chemicals; 2',4,4'-trichloro,2-hydroxydiphenyl ether (triclosan); and 3,7,11-trimethyldodeca-2,6,10-trienol (farnesol).

Aerosol compositions are comprised of a propellant and a base. An aerosol "base" composition is considered to be all the components of the aerosol composition minus the propellant.

It should be noted the propellants, even when liquefied, are not liquids according to the present invention, as they have boiling points below 20°C at atmospheric pressure.

Typically, the aerosol propellant is a liquefied hydrocarbon or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is especially preferred to employ liquefied hydrocarbon gases, and especially C₃ to C₆ hydrocarbons, including propane, butane, isobutane, pentane and isopentane and mixtures of two or more thereof. Preferred propellants are isobutane, isobutane/propane, butane/propane and mixtures of propane, isobutane and butane.

Other propellants that can be contemplated include alkyl ethers, such as dimethyl ether or compressed non-reactive gasses such air, nitrogen or carbon dioxide.

The propellant is typically the major component of aerosol compositions, often comprising from 30 to 99% weight and preferably comprising from 50 to 95% by weight.

In preferred embodiments, compositions may also comprise a suspending agent, for example, a hydrophobically modified clay, such as disteardimonium hectorite (Bentone 38V), ex Elementis, typically at from 0.1 to 1.5% by weight.

Propylene carbonate may also be advantageously employed in compositions used in accordance with the present invention, typically at from 0.001 to 0.1 % by weight.

Further additional components that may also be included are colourants and preservatives at a conventional level, for example C₁-C₃ alkyl parabens.

### Examples

The following examples illustrate certain specific embodiments of the invention and do not limit the scope of the invention.

All amounts are percentages by weight, unless otherwise indicated.

The components/ingredients used in the current study are detailed below.
(1) Cyclopentasiloxane, PMX-0245, ex Xiameter from Dow Corning.
(2) C12-15 alkyl benzoate, Finsolv TN, ex Innospec.
(3) Propylene carbonate.
(4) Potassium alum ex Sigma-Aldrich dried at 65°C [to 14-18% H₂0] and jet milled to give a particle size (D50) of 38-45 micron.
(5) Anhydrous calcium chloride - 7% water (by weight), ex Sigma-Aldrich, jet milled to give a particle size (D50) of 20-35 micron.
(6) Disteardimonium hectorite, Bentone 38V, ex Elementis.
(7) AP40, ex HARP.
(8) Dicapryly ether, Cetiol OE, ex BASF.
(9) Octyl dodecanol, Eutanol G, ex Cognis.
(10) Triglyceride oil, Sunflower seed oil, Florasun 90 ex Floratech.

In a first series of experiments, the interaction between dried calcium chloride and a range of fragrances comprising solvent (F1 to F9 as indicated in Table 1) was investigated by measuring the temperature rise on mixing. The temperature rise is indicative of an exothermic reaction between the calcium chloride and the solvent in the fragrance. It is hypothesised that strong exothermic reaction between the calcium chloride and fragrance solvent leads to rheological and/or homogeneity changes and consequential problems in cosmetic compositions according to the invention.

In the first series of experiments, 16g of the indicated fragrance was placed in a glass vial and the temperature noted. 4g of dried calcium chloride (17) was then added and the vial shaken to thoroughly mix the components. With the calcium chloride still suspended in the fragrance, the temperature was re-measured. The temperature rise observed is indicated in Table 1. Temperature rises of 3°C or greater were found to correlate with the consequential problems as described above and as illustrated in the further examples to follow. Such rises were observed when the fragrance plus solvent combination did not comprise one or more components having and a LogP of greater than 5.5 and/or comprised 3% or greater of dipropyleneglycol (DPG).

**Table 1**

| Fragrance-solvent | % of fragrance-solvent pre-mix | | Temperature rise on mixing (°C) |
|---|---|---|---|
| | Components of LogP >5.5* | DPG | |
| F1 | 22.4** | <0.1% | 1.5 |
| F2 | 25** | <0.1% | 1.5 |
| F3 | 25.4** | <0.1% | 1.0 |
| F4 | ∼0.3 | 0.2% | 2.0 |
| F5 | 35 | 3.0% | 5.0 |
| F6 | ∼0 | 5.4% | 8.0 |
| F7 | ∼0 | 25.0% | 13.0 |
| F8 | unknown | 15.0% | 18.0 |
| F9 | 0.8 | 27.7% | 21.0 |

| | | | |
|---|---|---|---|
| * And also vapour pressure of less than 0.001 mm Hg at 25°C ** Entirely isopropyl myristate (vapour pressure 0.00033 mm Hg at 25°C). | | | |

In a second series of experiments, the cosmetic compositions indicated in Table 2 were prepared using the fragrance-solvent combinations as indicated in Table 1. These compositions were prepared by taking the fragrance-solvent pre-mix and mixing it with silicone oil (1) and ester oil (2) to act as liquid carrier material materials and with a suspending agent (6), each component being added with shear. Dried calcium chloride (5) and dried potassium alum (4) were then added with shear. The resulting "base" compositions were then placed in glass aerosol containers which were closed with a standard valve/valve cup and gassed with liquefied propellant (7) to give the antiperspirant aerosol compositions as indicated.

**Table 2**

| **Example:** | **1** | **2** | **3** | **A** |
|---|---|---|---|---|
| **Component:** | | | | |
| Silicone oil (1) | 5.37 | 5.37 | 5.37 | 5.37 |
| Ester oil (2) | 2.0 | 2.0 | 2.0 | 2.0 |
| F1 | 1.0 | | | |
| F2 | | 1.0 | | |
| F4 | | | 1.0 | |
| F8 | | | | 1.0 |
| Dried potassium alum (4) | 2.34 | 2.34 | 2.34 | 2.34 |
| Dried calcium chloride (5) | 1.79 | 1.79 | 1.79 | 1.79 |
| Suspending agent (6) | 0.50 | 0.50 | 0.50 | 0.50 |
| Propellant (7) | To 100 | To 100 | To 100 | To 100 |

Examples 1 to 3 were prepared in accordance with the method of the present invention and had acceptable stability properties - the solid components did slowly sediment over time (30 to 60 seconds), but they remained re-dispersible. Comparative Example A, on the other hand, was prepared with a fragrance comprising 15% DPG and had unacceptable stability properties - the dried calcium chloride (5) and dried alum (4) settled very rapidly (2 to 5 seconds) and did not remain sufficiently re-dispersible.

In a further series of experiments, the cosmetic compositions indicated in Table 3 were prepared using the fragrance-solvent combinations as indicated in Table 1. These compositions were prepared using the same method as used for those of Table 2, but with propylene carbonate being adding to the mixture before the dried calcium chloride (5) and dried potassium alum (4).

**Table 3**

| **Example:** | **4** | **B** | **C** | **D** |
|---|---|---|---|---|
| **Component:** | | | | |
| Silicone oil (1) | 5.67 | 5.57 | 5.67 | 5.67 |
| Ester oil (2) | 2.0 | 2.0 | 2.0 | 2.0 |
| Propylene carbonate | 0.10 | 0.10 | 0.10 | 0.10 |
| F3 | 0.6 | | | |
| F6 | | 0.7 | | |
| F7 | | | 0.6 | |
| F9 | | | | 0.6 |
| Dried potassium alum (4) | 2.34 | 2.34 | 2.34 | 2.34 |
| Dried calcium chloride (5) | 1.79 | 1.79 | 1.79 | 1.79 |
| Suspending agent (6) | 0.50 | 0.50 | 0.50 | 0.50 |
| Propellant (7) | To 100 | To 100 | To 100 | To 100 |

Example 4 was prepared in accordance with the method of the present invention and had acceptable stability properties - the solid components did slowly sediment over time (30 to 60 seconds), but they remained re-dispersible. Comparative Examples B, C, and D, on the other hand, had unacceptable stability properties - the dried calcium chloride (5) and dried alum (4) settled very rapidly (2 to 5 seconds) and did not remain sufficiently re-dispersible.

In a further series of experiments, the cosmetic compositions indicated in Table 4 were prepared using the fragrance-solvent combinations as indicated in Table 1. These compositions were prepared using the same method as used for those of Table 3.

**Table 4**

| **Example:** | **5** | **6** | **7** | **E** | **F** |
|---|---|---|---|---|---|
| **Component:** | | | | | |
| Silicone oil (1) | 5.27 | 5.27 | 5.27 | 5.27 | 5.27 |
| Ester oil (2) | 2.0 | 2.0 | 2.0 | 2.0 | 2.0 |
| Propylene carbonate | 0.10 | 0.10 | 0.10 | 0.10 | 0.10 |
| F1 | 1.0 | | | | |
| F2 | | 1.0 | | | |
| F4 | | | 1.0 | | |
| F5 | | | | 1.0 | |
| F8 | | | | | 1.0 |
| Dried potassium alum (4) | 2.34 | 2.34 | 2.34 | 2.34 | 2.34 |
| Dried calcium chloride (5) | 1.79 | 1.79 | 1.79 | 1.79 | 1.79 |
| Suspending agent (6) | 0.50 | 0.50 | 0.50 | 0.50 | 0.50 |
| Propellant (7) | To 100 | To 100 | To 100 | To 100 | To 100 |

Examples 5, 6 and 7 were prepared in accordance with the method of the present invention and had acceptable stability properties - the solid components did slowly sediment over time (30 to 60 seconds), but they remained re-dispersible. Comparative Examples E and F, on the other hand, had unacceptable stability properties - the dried calcium chloride (5) and dried alum (4) settled very rapidly (2 to 5 seconds) and did not remain sufficiently re-dispersible.

In a further series of experiments, the aerosol base compositions indicated in Table 5 were prepared using the fragrance-solvent combinations as indicated in Table 1. These compositions were prepared by taking the fragrance-solvent pre-mix and mixing it with silicone oil (1) and ester oil (2) to act as liquid carrier material materials and with a suspending agent (6), each component being added with shear. Dried calcium chloride (5) and dried potassium alum (4) were then added with shear.

**Table 5**

| **Example:** | **8** | **9** | **10** | **11** |
|---|---|---|---|---|
| **Component:** | | | | |
| Silicone oil (1) | 41.31 | 41.31 | 44.38 | 41.31 |
| Ester oil (2) | 15.38 | 15.38 | 15.38 | 15.38 |
| F1 | 7.69 | | | |
| F2 | | 7.69 | | |
| F3 | | | 4.62 | |
| F4 | | | | 7.69 |
| Dried potassium alum (4) | 18.00 | 18.00 | 18.00 | 18.00 |
| Dried calcium chloride (5) | 13.77 | 13.77 | 13.77 | 13.77 |
| Suspending agent (6) | 3.85 | 3.85 | 3.85 | 3.85 |
| Stability | Viscosity remains acceptable for at least 33 days | | Composition thickens (>20000cP) within 11 days | Composition thickens (>20000cP) within 7 days |

Examples 8 and 9 exhibited excellent stability over time, their viscosity remaining within acceptable boundaries (less than 10,000 cP) for at least 33 days. Examples 10 and 11 were less successful, with viscosities reaching more than 20,000 cPs within 11 days and 7 days, respectively. It is hypothesised that the slight problem with these compositions lay in the nature of the fragrance-solvent pre-mix used in their preparation. Example 10 employed fragrance F3, which whilst comprising 25.4% isopropyl myristate, also comprised 12.5% butyl citrate and 3.7% diethyl phthalate, both of these latter being components having a logP of less than 5.5. An even less satisfactory performance was found with Example 11, which employed fragrance F4 in its manufacture. Fragrance F4 comprised components having a logP of greater than 5.5 and a vapour pressure at 25°C of less than 0.001 mm Hg at a level of only about 0.3%.

It may be noted that Example compositions 10 and 11 make perfectly acceptable base compositions for aerosol compositions provided that they are not stored for too long before they are gassed.

In a further series of experiments, the aerosol compositions indicated in Table 6 were prepared using the fragrance-solvent combinations F2 and F7 indicated in Table 1. These compositions were prepared by first mixing the oils indicated, the propylene carbonate, and the suspending agent, each component being added with shear. Dried calcium chloride and dried potassium alum were then added, also with shear. The resulting compositions were then placed in glass aerosol containers together with the fragrance-solvent mixture (F2 or F7). The glass aerosol containers were then closed with a standard valve/valve cup and gassed with liquefied propellant to give the antiperspirant aerosol compositions as indicated.

**Table 6**

| **Example:** | **12** | **13** | **14** | **15** | **G** | **H** | **I** | **J** |
|---|---|---|---|---|---|---|---|---|
| **Component:** | | | | | | | | |
| Silicone oil (1) | 7.27 | 5.27 | 5.27 | 5.27 | 7.27 | 5.27 | 5.27 | 5.27 |
| Ester oil (2) | | | 1.00 | 1.00 | | | 1.00 | 1.00 |
| Cetiol OE (8) | | 2.00 | | | | 2.00 | | |
| Octyl dodecanol (9) | | | 1.00 | | | | 1.00 | |
| Triglyceride oil (10) | | | | 1.00 | | | | 1.00 |
| Propylene carbonate (3) | 0.10 | 0.1 | 0.10 | 0.1 | 0.10 | 0.1 | 0.10 | 0.1 |
| F2 | 1.00 | 1.00 | 1.00 | 1.00 | | | | |
| F7 | | | | | 1.00 | 1.00 | 1.00 | 1.00 |
| Dried potassium alum (4) | 2.34 | 2.34 | 2.34 | 2.34 | 2.34 | 2.34 | 2.34 | 2.34 |
| Dried calcium chloride (5) | 1.79 | 1.79 | 1.79 | 1.79 | 1.79 | 1.79 | 1.79 | 1.79 |
| Suspending agent (6) | 0.50 | 0.50 | 0.50 | 0.50 | 0.50 | 0.50 | 0.50 | 0.50 |
| Propellant (7) | To 100 | To 100 | To 100 | To 100 | To 100 | To 100 | To 100 | To 100 |

Examples 12, 13, 14 and 15 had acceptable stability properties: whilst the solid components did slowly sediment over time (30 to 60 seconds), they remained re-dispersible. Comparative Examples G, H, I and J had unacceptable stability properties: the dried calcium chloride and dried alum settled rapidly (in less than 10 to15 seconds) and did not remain sufficiently re-dispersible.

## Claims

1. A method of manufacture of an anhydrous cosmetic composition, having less than 2% by weight free water, said method comprising the mixing of dried calcium chloride and a dried alum salt with liquid carrier material and fragrance comprising a solvent to give a base composition, followed by gassing of the base composition with a propellant to give an aerosol composition, wherein the fragrance and the solvent therein are pre-mixed and this mixture:
i). comprises one or more components having a logP of greater than 5.5 and a vapour pressure at 25°C of less than 0.001 mm Hg (0.1333 Pa);
ii). does not comprise dipropyleneglycol at 3% or greater of this mixture;
the liquid carrier material including all the liquid components of the composition, excluding any liquid fragrance and solvent comprised therein.

2. A method according to claim 1, wherein a suspending agent is also mixed into the base composition prior to gassing.

3. A method according to claim 2, wherein the suspending agent is hydrophobically modified clay.

4. A method according to any of the preceding claims, wherein the pre-mixed fragrance and solvent does not comprise dipropyleneglycol at 1% or greater.

5. A method according to any of the preceding claims, wherein the pre-mixed fragrance and solvent comprises one or more components having a logP of greater than 5.5 and a vapour pressure at 25°C of less than 0.001 mm Hg at a level of 20% or greater by weight of said pre-mix.

6. A method according to any of the preceding claims, wherein the one or more components having a logP of greater than 5.5 and a vapour pressure at 25°C of less than 0.001 mm Hg (0.1333 Pa) is predominately comprised of isopropyl esters of myristic acid, lauric acid, and/or palmitic acid.

7. A method according to claim 6, wherein the one or more components having a logP of greater than 5.5 and a vapour pressure at 25°C of less than 0.001 mm Hg (0.1333 Pa) is predominately comprised of isopropyl myristate.

8. A method according to claim 7, wherein the one or more components having a logP of greater than 5.5 and a vapour pressure at 25°C of less than 0.001 mm Hg (0.1333 Pa) is comprised of isopropyl myristate to a level of 90% by weight or greater.

9. A method according to any of the preceding claims, wherein the dried calcium chloride is dried to a water content of less than 8% by weight prior to being mixed into the composition.

10. A method according to any of preceding claims, wherein the propellant is a liquefied hydrocarbon gas.

11. A method according to any of claims 2 to 10, wherein the dried alum salt is dried potassium aluminium sulphate.

12. A method according to claim 11, wherein the dried calcium chloride is added to the mixture at a molar ratio to the dried potassium aluminium sulphate of at least 2:1.

13. A method according to any of the preceding claims, wherein the liquid carrier material serves to comprise from 20% to 70% by weight of the composition, excluding the propellant that is also present in the final composition.

## Patentansprüche

1. Verfahren zur Herstellung einer wasserfreien kosmetischen Zusammensetzung, die weniger als 2 Gewichts-% freies Wasser aufweist, wobei das Verfahren das Mischen von getrocknetem Calciumchlorid und einem getrockneten Alaunsalz mit flüssigem Trägermaterial und Duftstoff, der ein Lösungsmittel umfasst, einbezieht, um eine Grundzusammensetzung zu liefern, gefolgt vom Begasen der Grundzusammensetzung mit einem Treibmittel, um eine Aerosolzusammensetzung zu liefern, wobei der Duftstoff und das darin enthaltene Lösungsmittel vorgemischt werden und diese Mischung
i) ein oder mehrere Bestandteile mit einem logP von mehr als 5,5 und einem Dampfdruck bei 25°C von weniger als 0,001 mm Hg (0,1333 Pa) umfasst,
ii) nicht Dipropylenglycol in 3% oder mehr dieser Mischung umfasst,
wobei das flüssige Trägermaterial sämtliche flüssigen Bestandteile der Zusammensetzung einbezieht, wobei flüssiger Duftstoff und darin enthaltenes Lösungsmittel sämtlich ausgeschlossen sind.

2. Verfahren nach Anspruch 1, wobei vor dem Begasen auch ein Suspendiermittel in die Grundzusammensetzung eingemischt wird.

3. Verfahren nach Anspruch 2, wobei das Suspendiermittel hydrophob modifizierter Ton ist.

4. Verfahren nach irgendeinem der vorhergehenden Ansprüche, wobei Duftstoff und Lösungsmittel, vorgemischt, kein Dipropylenglycol in 1% oder mehr umfassen.

5. Verfahren nach irgendeinem der vorhergehenden Ansprüche, wobei Duftstoff und Lösungsmittel, vorgemischt, ein oder mehrere Bestandteile mit einem logP von mehr als 5,5 und einem Dampfdruck bei 25°C von weniger als 0,001 mm Hg in einem Anteil von 20% oder mehr, in Gewicht der Vormischung, aufweisen.

6. Verfahren nach irgendeinem der vorhergehenden Ansprüche, wobei der eine oder die mehreren Bestandteile mit einem logP von mehr als 5,5 und einem Dampfdruck bei 25°C von weniger als 0,001 mm Hg (0,1333 Pa) überwiegend von Isopropylestern der Myristinsäure, Laurinsäure und/oder Palmitinsäure umfasst werden.

7. Verfahren nach Anspruch 6, wobei der eine oder die mehreren Bestandteile mit einem logP von mehr als 5,5 und einem Dampfdruck bei 25°C von weniger als 0,001 mm Hg (0,1333 Pa) überwiegend von Isopropylmyristat umfasst werden.

8. Verfahren nach Anspruch 7, wobei der eine oder die mehreren Bestandteile mit einem logP von mehr als 5,5 und einem Dampfdruck bei 25°C von weniger als 0,001 mm Hg (0,1333 Pa) in einem Anteil von 90 Gewichts-% oder mehr von Isopropylmyristat umfasst werden.

9. Verfahren nach irgendeinem der vorhergehenden Ansprüche, wobei das getrocknete Calciumchlorid bis auf einen Wassergehalt von weniger als 8 Gewichts-% getrocknet wird, bevor es der Zusammensetzung zugemischt wird.

10. Verfahren nach irgendeinem der vorhergehenden Ansprüche, wobei das Treibmittel ein verflüssigtes Kohlenwasserstoffgas darstellt.

11. Verfahren nach irgendeinem der Ansprüche 2 bis 10, wobei das getrocknete Alaunsalz getrocknetes Kaliumaluminiumsulfat darstellt.

12. Verfahren nach Anspruch 11, wobei das getrocknete Calciumchlorid in einem Molverhältnis von mindestens 2:1 zu dem getrockneten Kaliumaluminiumsulfat gegeben wird.

13. Verfahren nach irgendeinem der vorhergehenden Ansprüche, wobei das flüssige Trägermaterial dazu dient, 20 bis 70 Gewichts-% der Zusammensetzung zu umfassen, wobei das Treibmittel, das auch in der endgültigen Zusammensetzung vorliegt, ausgeschlossen ist.

## Revendications

1. Procédé de fabrication d'une composition cosmétique anhydre, ayant moins de 2 % en masse d'eau libre, ledit procédé comprenant le mélange de chlorure de calcium séché et d'un sel d'alun séché avec un matériau de support liquide et un parfum comprenant un solvant pour fournir une composition de base, suivi par un gazage de la composition de base avec un agent de propulsion pour fournir une composition d'aérosol, où le parfum et le solvant dans celle-ci sont pré-mélangés et ce mélange :
i). comprend un ou plusieurs constituants ayant un logP supérieur à 5,5 et une pression de vapeur à 25°C inférieure à 0,001 mm Hg (0,1333 Pa) ;
ii). ne comprend pas de dipropylèneglycol à 3 % ou plus de ce mélange ;
le matériau de support liquide comprenant tous les constituants liquides de la composition, à l'exclusion de tout parfum et solvant liquides compris dans celui-ci.

2. Procédé selon la revendication 1, dans lequel un agent de suspension est également mélangé dans la composition de base avant le gazage.

3. Procédé selon la revendication 2, dans lequel l'agent de suspension est de l'argile hydrophobiquement modifiée.

4. Procédé selon l'une quelconque des revendications précédentes, dans lequel le parfum et le solvant pré-mélangés ne comprennent pas de dipropylèneglycol à 1 % ou plus.

5. Procédé selon l'une quelconque des revendications précédentes, dans lequel le parfum et le solvant pré-mélangés comprennent un ou plusieurs constituants ayant un logP supérieur à 5,5 et une pression de vapeur à 25°C inférieure à 0,001 mm Hg à une teneur de 20 % ou plus en masse dudit pré-mélange.

6. Procédé selon l'une quelconque des revendications précédentes, dans lequel les un ou plusieurs constituants ayant un logP supérieur à 5,5 et une pression de vapeur à 25°C inférieure à 0,001 mm Hg (0,1333 Pa) sont de manière prédominante constitués d'esters isopropyliques d'acide myristique, d'acide laurique, et/ou d'acide palmitique.

7. Procédé selon la revendication 6, dans lequel les un ou plusieurs constituants ayant un logP supérieur à 5,5 et une pression de vapeur à 25°C inférieure à 0,001 mm Hg (0,1333 Pa) sont de manière prédominante constitués de myristate d'isopropyle.

8. Procédé selon la revendication 7, dans lequel les un ou plusieurs constituants présentant un logP supérieur à 5,5 et une pression de vapeur à 25°C inférieure à 0,001 mm Hg (0,1333 Pa) sont constitués de myristate d'isopropyle à une teneur de 90 % en masse ou supérieure.

9. Procédé selon l'une quelconque des revendications précédentes, dans lequel le chlorure de calcium séché est séché jusqu'à une teneur en eau inférieure à 8 % en masse avant d'être mélangé dans la composition.

10. Procédé selon l'une quelconque des revendications précédentes, dans lequel l'agent de propulsion est un gaz hydrocarboné liquéfié.

11. Procédé selon l'une quelconque des revendications 2 à 10, dans lequel le sel d'alun séché est du sulfate de potassium aluminium séché.

12. Procédé selon la revendication 11, dans lequel le chlorure de calcium séché est ajouté au mélange à un rapport molaire par rapport au sulfate de potassium aluminium séché d'au moins 2:1.

13. Procédé selon l'une quelconque des revendications précédentes, dans lequel le matériau de support liquide sert à comprendre de 20 % à 70 % en masse de la composition, excluant l'agent de propulsion qui est également présent dans la composition finale.
